# EUROPÄISCHE PATENTSCHRIFT

(11) **EP 1 547 099 B1**
(45) Veröffentlichungstag und Bekanntmachung des Hinweises auf die Patenterteilung: **09.08.2006**
(21) Anmeldenummer: 02750950.4
(22) Anmeldetag: 18.05.2002
(51) Int. Cl.: G21K 5/04, B05D 3/06

(54) **Bestrahlungsvorrichtung und Verfahren zu ihrem Betrieb**
Radiation device and method for its operation
Dispositif d'émission de rayonnement et sa méthode de fonctionnement

(30) Priorität: 26.05.2001 DE 10125770
(43) Veröffentlichungstag der Anmeldung: 29.06.2005
(73) Patentinhaber: arccure technologies GmbH, 59557 Lippstadt (DE)
(72) Erfinder: SCHWEITZER, Bert, 41352 Korschenbroich (DE)
(74) Vertreter: Kohlmann, Kai
(86) Internationale Anmeldenummer: PCT/EP2002/005519
(87) Internationale Veröffentlichungsnummer: WO 2002/097828

(56) Entgegenhaltungen:
- WO-A-98/54525
- DE-A- 19 916 474
- US-A- 4 015 340

## Beschreibung

Die Erfindung betrifft eine Bestrahlungsvorrichtung zur Bestrahlung von Objekten mit insbesondere ultravioletter und/oder infraroter und/oder sichtbarer elektromagnetischer Strahlung, mit einem Gehäuse, das eine auf das zu bestrahlende Objekt ausgerichtete Austrittsöffnung für die elektromagnetische Strahlung aufweist, mindestens einer in dem Gehäuse angeordneten langgestreckten Strahlungsquelle für die elektromagnetische Strahlung, wobei die Bestrahlungsvorrichtung mindestens einen Zuführungskanal und mindestens ein Abzugskanal für Kühlgas aufweist, die Kanäle jeweils eine sich in Richtung der langgestreckten Strahlungsquelle über deren Länge erstreckende Kühlgasöffnung aufweisen wobei die Strahlungsquelle zwischen dem Zuführungskanal und dem Abzugskanal angeordnet ist und mindestens eine Gegenfläche die Strahlungsquelle unter Ausbildung eines sich in Richtung der Kühlgasöffnung des Abzugskanals verjüngenden Spaltes teilweise umgibt.

Bestrahlungsvorrichtungen, insbesondere für W-Licht kommen in der photochemischen Beeinflussung von Bestrahlungsobjekten zur Anwendung. Wichtige Anwendungen sind die Aushärtung von Druckfarben, Klebstoffen und Beschichtungen sowie die Sterilisation und die medizinische Bestrahlung. Insbesondere im Bereich der Beschichtung von Holzplatten und Fußbodenbelägen werden UV-Bestrahlungsvorrichtungen mit sehr hohen Bestrahlungsleistungen eingesetzt. Die Bestrahlungsbreite kann bis zu mehreren Metern betragen.

Als Strahlungsquellen in UV-Bestrahlungsvorrichtungen kommen vor allem Gasentladungslampen zum Einsatz, in denen durch das Verdampfen von Metallen ein Plasma erzeugt wird. Die Lampen bestehen dabei im wesentlichen aus einem röhrenförmigen Glaskörper, zwei Elektroden, zwei Folieneinschmelzungen sowie zwei Sockeln. Je nach Lampentyp betragen die Betriebstemperaturen am Glaskörper zwischen 700°C und 900°C.

Alle bekannten langgestreckten UV-Bestrahlungsvorrichtungen weisen eine an beiden Enden aufgehängte Strahlungsquelle auf, die teilweise von einem Reflektor umgeben sein kann.

Die Strahlungsquellen sind derart gestaltet, dass die vom Glas absorbierte Energie durch freie Konvektion und durch Strahlung abgegeben wird. Ein Gleichgewicht zwischen der absorbierten und der abgegebenen Energiemenge würde sich bei einer Temperatur des Glaskörpers von etwa 800°C einstellen. In der Praxis behindern aber die Reflektoren und das Gehäuse der UV-Bestrahlungsvorrichtung diesen Zustand. Es kommt zu Reflexion von Wärmestrahlung und teilweise sogar zu Hitzestaus in der Nähe der Strahlungsquelle.

Um dieses Problem zu lösen wird versucht, durch verbesserte Luftkühlsysteme die Temperatur der Strahlungsquelle in dem optimalen Betriebsbereich einzustellen.

Aus der DE 199 16 474 Al ist eine Bestrahlungsvorrichtung mit einer langgestreckten Strahlungsquelle zur Bestrahlung von Objekten mit UV-Strahlung bekannt, deren Gehäuse eine auf das zu bestrahlende Objekt ausgerichtete Austrittsöffnung aufweist. Zur Wärmeabfuhr weist die Vorrichtung ein Kühlsystem auf, in welchem ein Kühlgasstrom zur Umlaufkühlung der Strahlungsquelle im Gehäuseinneren mittels Gebläse zwangsweise umgewälzt und über einen Wärmetauscher rückgekühlt wird. Das Gebläse wird durch mehrere über die Länge der Strahlungsquelle verteilt angeordnete Radiallüfter gebildet, deren Lüfterachsen senkrecht zur Strahlungsquelle ausgerichtet sind. Das über einen seitlichen Kanal einströmende Kühlgas umspült zunächst den Mantel der Strahlungsquelle und strömt dann über einen Längsspalt im Scheitelbereich des oberhalb der Strahlungsquelle angeordneten Reflektors in eine dem Gebläse vorgeschaltete Ansaugkammer.

Nachteilig bei diesem Kühlsystem ist, dass mehrere über die Länge der Strahlungsquelle verteilt angeordnete Radiallüfter zur wirksamen Kühlung erforderlich sind. Außerdem kann die aus deren senkrechter Anordnung resultierende Bauhöhe den Einsatz der Bestrahlungsvorrichtung unter beengten Platzverhältnissen erschweren oder sogar ausschließen.

Ausgehend von diesem Stand der Technik liegt der Erfindung die Aufgabe zugrunde, eine besonders effektive Kühlung für die langgestreckte Strahlungsquelle zu schaffen.

Diese Aufgabe wird bei einer Bestrahlungsvorrichtung der eingangs erwähnten Art dadurch gelöst, dass jeder Kanal von einem Innenraum des Gehäuses der Bestrahlungsvorrichtung durch ein Strömungssieb getrennt ist, dass einer der Kanäle integraler Bestandteil einer langgestreckten Barriere ist, die den direkten Strahlengang von der Strahlungsquelle auf das zu bestrahlende Objekt zumindest teilweise ausblendet und die Barriere im Querschnitt bogenförmige Reflektorelemente besitzt, die zusammen eine langgestreckte Mulde bilden, die die Strahlungsquelle teilweise an ihrer Unterseite umgibt.

Der sich verjüngende Spalt zwischen Strahlungsquelle und Gegenfläche erhöht die Strömungsgeschwindigkeit des Kühlgases vom Eintritt in den Spalt bis zum Eintritt in den Abzugskanal. Die Geschwindigkeitszunahme verhindert, dass sich die Strömung von der Strahlungsquelle ablöst und stellt damit sicher, dass auch die dem Zuführungskanal abgewandet Seite der Strahlungsquelle ständig von Kühlgas überstrichen wird.

Vorzugsweise ist der Abzugskanal von der Austrittsöffnung für die Strahlung betrachtet vor und der Zuführungskanal hinter der Strahlungsquelle angeordnet. Die Kühlung der Strahlungsquelle schafft eine Entlastung gegen Durchbiegung. Das in den Zuführungskanal vorzugsweise an seiner Stirnseite eingespeiste Kühlgas, insbesondere Luft, erzeugt in Verbindung mit dem Sieb einen gleichmäßigen Überdruck über die gesamte Länge der Strahlungsquelle. Das Sieb an dem Abzugskanal, der vorzugsweise an seiner Stirnseite mit einem Unterdruckerzeuger verbunden ist, vergleichmässigt den Unterdruck über die gesamte Länge der Strahlungsquelle.

Eine verbesserte Kühlung der Unterseite der Strahlungsquelle lässt sich erzielen, in dem insbesondere der Abzugskanal integraler Bestandteil der langgestreckten Barriere ist, die den direkten Strahlengang von der Strahlungsquelle auf das zu bestrahlende Objekt zumindest teilweise ausblendet, wobei jede Gegenfläche zur Ausbildung des sich verjüngenden Spaltes von der Oberfläche der Barriere oder von zwischen der Barriere und der Strahlungsquelle angeordneten Reflektoren gebildet wird. Die Oberfläche der Barriere oder der Reflektoren umgibt teilweise die Strahlenquelle unter Ausbildung des sich in Richtung der Kühlgasöffnung verjüngenden Spaltes. Der sich verjüngende Spalt zwischen Strahlungsquelle und Barriere erhöht die Strömungsgeschwindigkeit des Kühlgases vom Eintritt in den Spalt bis zum Eintritt in den Abzugskanal.

Alternativ kann der Abzugskanal von der Austrittsöffnung für die Strahlung betrachtet hinter der Strahlungsquelle angeordnet sein.

Selbst bei einer optimalen Kühlung der freihängenden, dass heißt lediglich endseitig gehaltenen Strahlungsquelle ist, dass ab einer kritischen elektrischen Energie in Kombination mit einer kritischen Baulänge die Temperatur des Glaskörpers so hoch wird, dass sich alle bekannten Strahlungsquellen mit der Schwerkraft verformen. Dieses Durchhängen ist bei allen Bestrahlungsquellen, nicht nur UV-Bestrahlungsquellen zu beobachten. Aufgrund von Kriechvorgängen des Glaskörpers, dessen Betriebstemperatur kurz vor dem plastischen Zustand des Materials liegt, verformen sich alle derartigen Strahlungsquellen mehr oder weniger schnell. Diese Eigenschaft wird bereits bei der Konzeption der Gehäuseform und dem Abstand zum zu bestrahlenden Objekt berücksichtigt. Wird die Verformung allerdings zu stark, so kann das in der Lampe entstandene Plasma punktuell in Kontakt zum Glaskörper kommen. Der Kontakt führt zu einer Überhitzung und damit zur Zerstörung des Glaskörpers der Bestrahlungsquelle.

Um eine UV-Bestrahlungsvorrichtung zu schaffen, die größere Bestrahlungsbreiten ohne eine Verformung der Strahlungsquelle ermöglicht, wird in einer Ausgestaltung der Erfindung vorgeschlagen, dass mindestens eine der Strahlungsquellen in der Bestrahlungsvorrichtung um ihre Längsachse drehbar angeordnet ist.

Die Drehung der Strahlungsquelle um ihre Längsachse gleicht den Einfluss der Schwerkraft auf die Kriechvorgänge im Material der Strahlungsquelle aus. Da sich die Betriebstemperaturen mit vertretbarem Kühlungsaufwand nur um eine gewisses Maß reduzieren lassen, basiert die Erfindung auf der Eliminierung der schädlichen Auswirkungen der auf die Strahlungsquelle einwirkenden Kräfte, in dem sich durch die Drehung die Richtung der an dem Material angreifenden Schwerkraft ständig verändert wird.

Bei freier Konvektion ist die Temperatur an der Oberseite der Strahlungsquelle höher als an deren Unterseite, da die Oberseite durch die nach oben strömende Luft stärker erwärmt wird. Dadurch kann es an der Oberseite zu lokalen Überhitzungen kommen, die die Strahlungsquelle zerstören können. Um diese Zerstörungen zu vermeiden, werden die Strahlungsquellen nach der kritischen Temperatur an der Oberseite ausgelegt. In dem die Lampe gedreht wird, kann sie bei höherer spezifischer Leistung betrieben werden, da stets wechselnde Bereiche den höchsten Temperaturen ausgesetzt sind.

Gemäß der Erfindung ist es möglich, dass in einer Bestrahlungsvorrichtung mehrere Strahlungsquellen achsparallel zueinander angeordnet sind, von denen wenigstens eine drehbar um ihre Längsachse angeordnet ist. Vorteilhafterweise sind jedoch sämtliche in der Bestrahlungsvorrichtung angeordneten Strahlungsquellen drehbar. In dem zuletzt genannten Fall sind die Strahlungsquellen vorteilhafterweise mit einem gemeinsamen Antrieb verbunden. Die Verbindung erfolgt beispielsweise über ein Riemen- oder ein Planentengetriebe.

Um Beschädigungen des regelmäßig aus Glas bestehenden Körpers der Strahlungsquelle zu vermeiden, werden die beiden Enden jeder drehbaren Strahlungsquelle in einer drehbar gelagerten Aufnahme gehalten und mindestens eine der beiden Aufnahmen jeder Strahlungsquelle ist mit einem Antrieb verbunden. Sind beide Aufnahmen einer Strahlungsquelle mit einem Antrieb verbunden, lässt sich eine Torsion des Körpers der Strahlungsquelle durch das Antriebsmoment weitgehend verhindern. Der Antrieb für die Drehung der Strahlungsquellen kann beispielsweise pneumatisch, elektrisch oder auch manuell erfolgen. Bei kurzen Strahlungsquellen bis etwa 2 m Länge genügt es jedoch, wenn der Antrieb lediglich an einem Ende angreift.

Insbesondere bei lediglich in einer vorgegebenen Richtung drehenden Strahlungsquellen ist es vorteilhaft, die für deren Betrieb erforderlichen Energie kontaktlos zuzuführen, beispielsweise über elektromagnetische Strahlung (Mikrowellenanregung). Alternativ ist eine herkömmliche Energieversorgung über Schleifkontakte möglich.

Bei Drehung der Strahlungsquelle mit regelmäßiger Richtungsänderung kann die elektrische Energie auch über flexible Kabel erfolgen, vorausgesetzt die Drehwinkel in entgegengesetzter Richtung halten sich die Waage.

Um ein Kräftegleichgewicht in dem Körper der Strahlungsquelle zu erzeugen, kann die Drehung der Strahlungsquelle in zeitlichen Abständen richtungsgeändert und / oder unterbrochen werden. Eine Unterbrechung der Drehung mit Drehrichtungsänderung ist zweckmäßig, wenn eine kontinuierliche Drehung unerwünscht ist.

Wird die Drehrichtung der Strahlungsquelle jeweils nach einer Teildrehung der Strahlungsquelle um mindestens 180 °geändert, so erfährt jeder Punkt des Körpers der Strahlungsquelle die angreifende Schwerkraft einmal als positiven und einmal als negativen Kraftvektor in annähernd gleicher Größe. Die bei einer langsamen Drehung auftretenden Verformungen der Strahlungsquelle stellen sich also immer wieder zurück. Vorteilhafte Drehzahlen der Strahlungsquelle liegen im Bereich von 0,1 bis 0,2 r/s.

Um die Verformungen im Gleichgewicht zu halten, sollte die Drehzahl der Strahlungsquelle nach dem Anfahren konstant gehalten werden. Dadurch wird gewährleistet, dass jeder Punkt des Körpers der Strahlungsquelle für die gleiche Dauer derselben Schwerkraft sowohl in negativer als auch in positiver Richtung ausgesetzt wird.

Nachfolgend wird die Erfindung anhand der Zeichnungen näher erläutert. Es zeigen:
- **Fig. 1 a - b**: eine Vorderansichten und eine Seitenansicht einer UV-Bestrahlungsvorrichtung in schematischer Darstellung, jedoch ohne Kühlvorrichtung,
- **Fig. 2 a, b**: eine Vorderansicht eines Ausführungsbeispiels einer erfindungsgemäßen UV-Bestrahlungsvorrichtung mit besonders wirksamer Kühlvorrichtung in schematischer Darstellung.

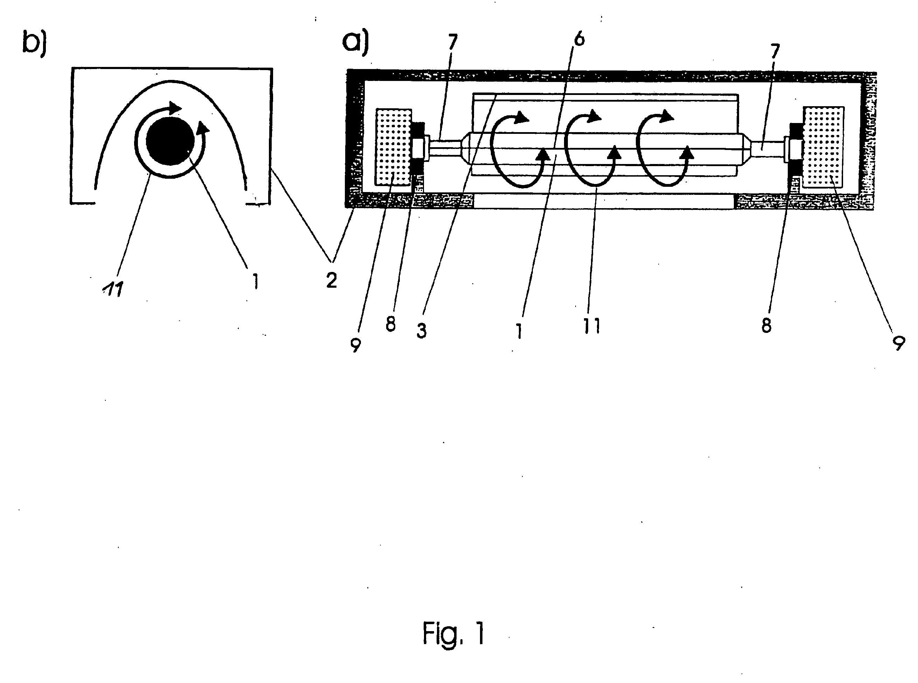

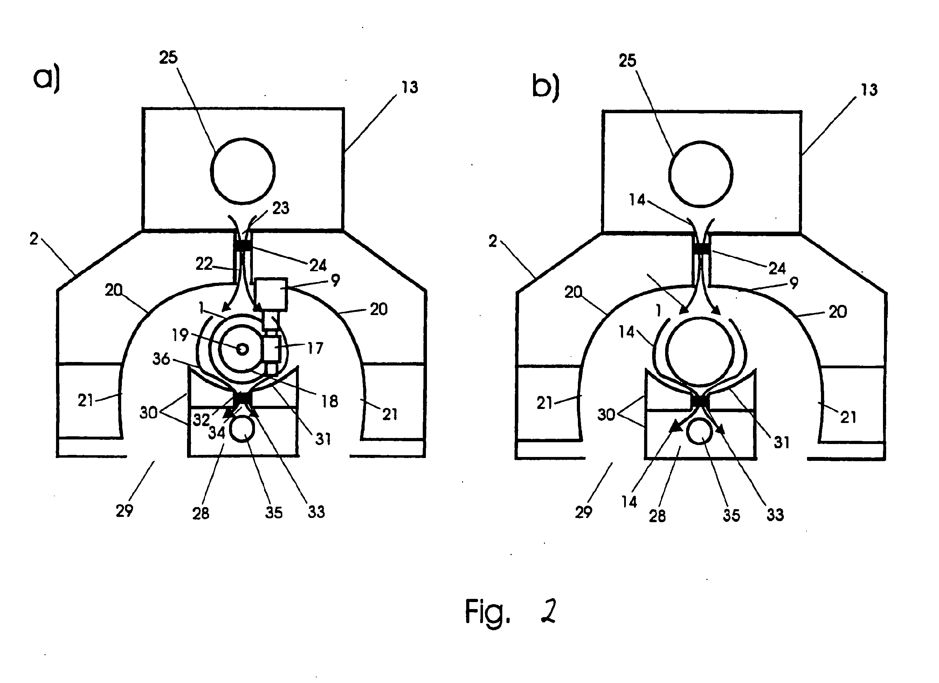

Fig. 1 a) zeigt der Übersichtlichkeit halber zunächst ohne die erfindungsgemäße Kühlung eine röhrenförmige, langgestreckte Strahlungsquelle 1, die innerhalb eines Gehäuses 2 zwischen einem Reflektor 3 und dem zu bestrahlenden Objekt angeordnet ist. Die Strahlungsquelle 1 ist um ihre Längsachse 6 drehbar in dem Gehäuse 2 der Bestrahlungsvorrichtung angeordnet. Eine vollständige Darstellung einer erfindungsgemäßen Vorrichtung mit drehbarer Strahlungsquelle (1) ergibt sich aus Figur 2 a). Die Enden 7 der Strahlungsquelle werden von Aufnahmen 8 gehalten, die drehbar gelagert sind. Der Antrieb für die Drehung der Strahlungsquelle 1 erfolgt über die beiden Antriebe 9 und das darin integrierte, nicht näher dargestellte Getriebe. Die durch den Pfeil 11 in den Figuren 1 a), b) angedeutete Drehrichtung der Strahlungsquelle ist mittels einer nicht dargestellten, an sich bekannten Umkehrsteuerung änderbar.

Eine erfindungsgemäße Bestrahlungsvorrichtung nach Figur 2 a) weist eine wirksame Kühlung für die Unter- und Oberseite der Strahlungsquelle 1 auf. Die in dem Beispiel als UV-Lampe ausgebildete Strahlungsquelle 1 ist in dem Gehäuse 2 drehbar angeordnet. Eine Schneckenwelle 17 des elektrischen Antriebs 9 kämmt mit einem Zahnrad 18, das in einer zentralen Öffnung 19 eines der Enden 7 der Strahlungsquelle 1 aufnimmt.

Der Reflektor 3 besteht aus zwei sich über die gesamte Länge der Bestrahlungsvorrichtung erstreckenden oberen Reflektorelementen 20 sowie zwei unteren Reflektorelementen 21. Die beiden oberen Reflektorelemente 20 enden im Bereich einer gedachten Längsmittelebene durch die Bestrahlungsvorrichtung im Abstand voneinander unter Ausbildung eines Lufteinlassschlitzes 22. Auf der Oberseite des Gehäuses 2 befindet sich ein Zuführungskanal 13 für die Kühlluft. Der Zuführungskanal 13 erstreckt sich zumindest über die gesamte Länge des Lufteinlassschlitzes 22. Er weist an seiner Unterseite eine Kühlluftöffnung 23 auf, die den Lufteinlassschlitz 22 überdeckt. Zwischen dem Lufteinlassschlitz 22 und der Kühlluftöffnung 23 befindet sich ein engmaschiges Strömungssieb 24.

An der rückwärtigen Stirnseite des Zuführungskanals 13 befindet sich ein Einlass 25, über den die Kühlluft in den Zuführungskanal 13 gelangt. Das Strömungssieb 24 vergleichmässigt den Druck der Kühlluft über die gesamte Länge des Lufteinlassschlitzes 22 und bewirkt damit eine gleichmäßige Kühlung der Strahlungsquelle über deren gesamte Länge.

Die üblicherweise auftretende Ablösung der Kühlluft von der Strahlungsquelle 1 wird bei der Bestrahlungsvorrichtung nach Figur 2a, b) wirksam durch einen zusätzlichen Abzugskanal 28 verhindert. Der Abzugskanal 28 befindet sich von der Austrittsöffnung 29 für die Strahlung betrachtet, vor der Strahlungsquelle 1 auf der gedachten Längs-Mittel-Ebene durch die Bestrahlungsvorrichtung. Der Abzugskanal 28 ist integraler Bestandteil einer insgesamt mit 30 bezeichneten, langgestreckten Barriere, die den direkten Strahlengang von der Strahlungsquelle 1 auf das zu bestrahlende Objekt zumindest teilweise ausblendet. Zu diesem Zweck besitzt die Barriere im Querschnitt bogenförmige Reflektorelemente 31, die zusammen eine langgestreckte Mulde bilden, die die Strahlungsquelle 1 teilweise an ihrer Unterseite umgibt.

Die voneinander beabstandeten Reflektorelemente 31 bilden einen Luftauslassschlitz 32 , der sich über die gesamte Länge der Strahlungsquelle 1 erstreckt. Der Luftauslassschlitz 32 ist durch ein weiteres Strömungssieb 33 von einer Kühlluftöffnung 34 des Abzugskanals 28 getrennt. Die Kühlluftöffnung 34 erstreckt sich ebenfalls über die gesamte Länge der Strahlungsquelle 1. Das Strömungssieb 33 bewirkt an dem Abzugskanal 28 ebenfalls eine Vergleichmäßigung der Druckverhältnisse, allerdings hier des in dem Abzugskanal 28 herrschenden Unterdrucks. Der Unterdruck wird beispielsweise durch eine an der rückwärtigen Stirnseite an einem Auslass 35 des Abzugskanals 28 angeschlossene, nicht dargestellte Vakuumpumpe erzeugt. Er kann jedoch auch durch ein Gebläse erzeugt werden, dessen Saugseite mit dem Abzugskanal und dessen Druckseite mit dem Zuführungskanal verbunden ist. In dem auf diese Weise geschlossenen Kreislauf wird die Kühlluft zusätzlich gefiltert und rückgekühlt.

Aus Figur 2 ist erkennbar, dass die die Oberfläche der Barriere bildenden Reflektorelemente 31 mit der Oberfläche der Strahlungsquelle 1 einen sich in Richtung des Abzugskanals 28 verjüngenden Spalt 36 ausbilden. Infolgedessen wird der zu beiden Seiten der Strahlungsquelle 1 in den Spalt 36 eintretende Kühlluftstrom 14 beschleunigt und damit dessen Ablösung von der Strahlungsquelle 1 an deren Unterseite wirksam verhindert. Trotz dieser effektiven Kühlung der Unterseite der Bestrahlungsquelle 1 durch den in die Barriere integrierten Abzugskanal 28 kommt es infolge der Wärmeaufnahme des Kühlluftstroms nach wie vor zu einer höheren Temperatur der Strahlungsquelle 1 an deren Unterseite. Obwohl diese Temperaturdifferenz deutlich geringer als bei einer Bestrahlungsvorrichtung ohne Kühlung der Unterseite, ist es zur weiteren Vergleichmäßigung der Temperaturen zweckmäßig, die Strahlungsquelle drehbar, wie insbesondere in Figur 1 a), b) und Figur 2 a) dargestellt, anzuordnen und während des Betriebs zu drehen.

Die beschriebene Kühlvorrichtung lässt sich jedoch auch in Bestrahlungsvorrichtungen mit statisch angeordneten Strahlungsquellen, wie in Fig. 2 b) dargestellt, mit Vorteil einsetzen.

### Bezugszeichenliste

| **Nr.** | **Bezeichnung** | **Nr.** | **Bezeichnung** |
|---|---|---|---|
| 1. | Strahlungsquelle | 19. | Öffnung |
| 2. | Gehäuse | 20. | Obere Reflektorelemente |
| 3. | Reflektor | 21. | Untere Reflektorelemente |
| 4. | | 22. | Lufteinlassschlitz |
| 5. | | 23. | Kühlluftöffnung |
| 6. | Längsachse | 24. | Strömungssieb |
| 7. | Enden | 25. | Einlass |
| 8. | Aufnahme | 26. | |
| 9. | Antrieb | | |
| 10. | | 28. | Abzugskanal |
| 11. | Pfeil | 29. | Austrittsöffnung |
| 12. | | 30. | Barriere |
| 13. | Zuführungskanal | 31. | Reflektorelemente |
| 14. | Kühlluftstrom | 32. | Luftauslassschlitz |
| | | 33. | Strömungssieb |
| 16. | | 34. | Kühlluftöffnung |
| 17. | Schneckenwelle | 35. | Auslass |
| 18. | Zahnrad | 36. | Spalt |

## Patentansprüche

1. Bestrahlungsvorrichtung zur Bestrahlung von Objekten mit elektromagnetischer Strahlung, mit einem Gehäuse, das eine auf das zu bestrahlende Objekt ausgerichtete Austrittsöffnung für die elektromagnetische Strahlung aufweist, mindestens einer in dem Gehäuse angeordneten langgestreckten Strahlungsquelle für die elektromagnetische Strahlung, wobei die Bestrahlungsvorrichtung mindestens einen Zuführungskanal (13) und mindestens ein Abzugskanal (28) für Kühlgas aufweist, die Kanäle jeweils eine sich in Richtung der langgestreckten Strahlungsquelle (1) über deren Länge erstreckende Kühlgasöffnung (23, 34) aufweisen wobei die Strahlungsquelle (1) zwischen dem Zuführungskanal (13) und dem Abzugskanal (28) angeordnet ist und mindestens eine Gegenfläche die Strahlungsquelle (1) unter Ausbildung eines sich in Richtung der Kühlgasöffnung (34) des Abzugskanals verjüngenden Spaltes (36) teilweise umgibt, **dadurch gekennzeichnet, dass**
- jeder Kanal von einem Innenraum des Gehäuses (2) der Bestrahlungsvorrichtung durch ein Strömungssieb (24, 33) getrennt ist und dass
- einer der Kanäle (13, 28) integraler Bestandteil einer langgestreckten Barriere(30) ist, die den direkten Strahlengang von der Strahlungsquelle (1) auf das zu bestrahlende Objekt zumindest teilweise ausblendet und dass
- die Barriere im Querschnitt bogenförmige Reflektorelemente (31) besitzt, die zusammen eine langgestreckte Mulde bilden, die die Strahlungsquelle (1) teilweise an ihrer Unterseite umgibt.

2. Bestrahlungsvorrichtung nach Anspruch 1, **dadurch gekennzeichnet, dass** jede Gegenfläche zur Ausbildung des sich verjüngenden Spaltes (36) von der Oberfläche der Barriere gebildet wird.

3. Bestrahlungsvorrichtung nach Anspruch 1 oder 2, **dadurch gekennzeichnet, dass** mindestens eine der Strahlungsquellen (1) in der Bestrahlungsvorrichtung um ihre Längsachse drehbar angeordnet ist.

4. Bestrahlungsvorrichtung nach Anspruch 3, **dadurch gekennzeichnet, dass** die beiden Enden (7) jeder drehbaren Strahlungsquelle (1) in einer drehbaren Aufnahme (8) gehalten werden und mindestens eine der beiden Aufnahmen (8) jeder Strahlungsquelle (1) mit einem Antrieb (9) verbunden ist.

5. Bestrahlungsvorrichtung nach Anspruch 3 oder 4, **dadurch gekennzeichnet, dass** mehrere Strahlungsquellen (1) mit einem gemeinsamen Antrieb (9) verbunden sind.

6. Bestrahlungsvorrichtung nach einem der Ansprüche 3 bis 5, **dadurch gekennzeichnet, dass** sie Mittel zur kontaktlosen Zufuhr der für den Betrieb der Strahlungsquelle (1) erforderlichen Energie aufweist.

7. Verfahren zum Betrieb einer Bestrahlungsvorrichtung nach einem der Ansprüche 3 bis 6, **dadurch gekennzeichnet, dass** die Drehung der Strahlungsquelle in zeitlichen Abständen richtungsgeändert und /oder unterbrochen wird.

8. Verfahren nach Anspruch 7, **dadurch gekennzeichnet, dass** die Drehrichtung jeweils nach einer Teildrehung der Strahlungsquelle um mindestens 180 °geändert wird.

9. Verfahren zum Betrieb einer Strahlungsvorrichtung nach einem der Ansprüche 3 bis 6, **dadurch gekennzeichnet, dass** die Strahlungsquelle ständig in einer vorgegebenen Richtung gedreht wird.

10. Verfahren nach einem der Ansprüche 7 bis 9, **dadurch gekennzeichnet, dass** für die Strahlungsquelle eine insbesondere konstante Drehzahl im Bereich von 0,1 bis 0,2 r/s gewählt wird.

## Claims

1. An irradiation device for irradiating objects with electromagnetic radiation, comprising a housing with an outlet opening for the electromagnetic radiation that is directed toward the object to be irradiated, and at least one elongate radiation source for the electromagnetic radiation that is arranged in the housing, wherein the irradiation device features at least one supply channel (13) and at least one discharge channel (28) for cooling gas and the channels respectively feature a cooling gas opening (23, 34) that extends in the direction of the elongate radiation source (1) over the length thereof, and wherein the radiation source (1) is arranged between the supply channel (13) and the discharge channel (28) and at least one opposite surface partially surrounds the radiation source (1) such that a gap (36) is formed that is tapered in the direction of the cooling gas opening (34) of the discharge channel,
**characterized in that**
- each channel is separated from an interior of the housing (2) of the irradiation device by a flow screen (24, 33), and **in that**
- one of the channels (13, 28) forms an integral component of an elongate barrier (30) that at least partially blanks out the direct beam path from the radiation source (1) to the object to be irradiated, and **in that**
- the cross section of the barrier resembles arc-shaped reflector elements (31) that collectively form an elongate trough that partially surrounds the radiation source (1) on its underside.

2. The irradiation device according to Claim 1, **characterized in that** each opposite surface for realizing the tapered gap (36) is formed by the surface of the barrier.

3. The irradiation device according to Claim 1 or 2, **characterized in that** at least one of the radiation sources (1) is arranged in the irradiation device such that it is rotatable about its longitudinal axis.

4. The irradiation device according to Claim 3, **characterized in that** both ends (7) of each rotatable radiation source (1) are held in a rotatable receptacle (8) and at least one of the two receptacles (8) of each radiation source (1) is connected to a drive (9).

5. The irradiation device according to Claim 3 or 4, **characterized in that** several radiation sources (1) are connected to a common drive (9).

6. The irradiation device according to one of Claims 3-5, **characterized in that** it features means for the contactless supply of the energy required for operating the radiation source (1).

7. A method for operating an irradiation device according to one of Claims 3-6, **characterized in that** the rotation of the radiation source changes its rotating direction and/or is interrupted within certain time intervals.

8. The method according to Claim 7, **characterized in that** the rotating direction is respectively changed after a partial rotation of the radiation source by at least 180°.

9. A method for operating a radiation device according to one of Claims 3-6, **characterized in that** the radiation source continuously rotates in a predetermined direction.

10. The method according to one of Claims 7-9, **characterized in that** a rotational speed, particularly a constant rotational speed in the range between 0.1 and 0.2 r/s, is chosen for the radiation source.

## Revendications

1. Dispositif d'irradiation pour l'irradiation d'objets avec un rayonnement électromagnétique, avec un boîtier, comportant un orifice orienté vers l'objet à irradier pour le rayonnement électromagnétique, avec au moins une source de rayonnement allongée disposée dans le boîtier pour le rayonnement électromagnétique, le dispositif de rayonnement comportant au moins un conduit d'alimentation (13) et au moins un conduit d'évacuation (28) pour du gaz de refroidissement, les conduits comportant chacun un orifice pour gaz de refroidissement (23, 24) s'étendant dans le sens de la source de rayonnement allongée (1), sur la longueur de cette dernière, la source de rayonnement (1) étant disposée entre le conduit d'alimentation (13) et le conduit d'évacuation (28) et au moins une surface antagoniste entourant partiellement la source de rayonnement (1) en formant une fente (36) se rétrécissant dans le sens de l'orifice pour gaz de refroidissement (34) du conduit d'évacuation,
**caractérisé en ce que**
- chaque conduit est séparé d'un espace intérieur du boîtier (2) du dispositif d'irradiation par un tamis d'écoulement (24, 33) et **en ce que**
- l'un des conduits (13, 28) est une partie intégrale d'une barrière allongée (30) qui diaphragme au moins partiellement la trajectoire directe du faisceau de la source de rayonnement (1) sur l'objet à irradier et **en ce que**
- la barrière comporte des éléments réflecteurs (31) de section transversale cintrée, qui forment en commun une cavité allongée qui entoure partiellement la source de rayonnement (1) sur sa face inférieure.

2. Dispositif d'irradiation selon la revendication 1, **caractérisé en ce que** chaque surface antagoniste pour la formation de la fente se rétrécissant (36) est formée par la surface de la barrière.

3. Dispositif d'irradiation selon la revendication 1 ou 2, **caractérisé en ce qu'**au moins l'une des sources de rayonnement (1) est disposée dans le dispositif d'irradiation de façon rotative autour de son axe longitudinal.

4. Dispositif d'irradiation selon la revendication 3, **caractérisé en ce que** les deux extrémités (7) de chaque source de rayonnement rotative (1) sont maintenues dans un logement rotatif (8) et **en ce qu'**au moins l'un des logements rotatifs (8) de chaque source de rayonnement (1) est relié avec un entraînement (9).

5. Dispositif d'irradiation selon la revendication 3 ou 4, **caractérisé en ce que** plusieurs sources de rayonnement (1) sont reliées avec un entraînement commun (9).

6. Dispositif d'irradiation selon l'une quelconque des revendications 3 à 5, **caractérisé en ce qu'**il comporte des moyens pour l'alimentation sans contact de l'énergie nécessaire pour l'exploitation de la source de rayonnement (1).

7. Procédé d'exploitation d'un dispositif d'irradiation selon l'une quelconque des revendications 3 à 6, **caractérisé en ce que** la rotation de la source de rayonnement change de sens et/ou est interrompue par intervalles de temps.

8. Procédé selon la revendication 7, **caractérisé en ce qu'**on change le sens de rotation d'au moins 180° chaque fois après une rotation partielle de la source de rayonnement.

9. Procédé d'exploitation d'un dispositif d'irradiation selon l'une quelconque des revendications 3 à 6, **caractérisé en ce qu'**on tourne constamment la source de rayonnement dans un sens prédéfini.

10. Procédé selon l'une quelconque des revendications 7 à 9, **caractérisé en ce qu'**on opte pour une vitesse de rotation notamment constante, dans la plage de 0,1 à 0,2 r/s pour la source de rayonnement.
